# EUROPÄISCHE PATENTSCHRIFT

(11) **EP 4 447 876 B1**
(45) Veröffentlichungstag und Bekanntmachung des Hinweises auf die Patenterteilung: **26.11.2025**
(21) Anmeldenummer: 22839173.6
(22) Anmeldetag: 14.12.2022
(51) Int. Cl.: A61F 2/64, A61F 2/70, B25J 9/00, A61F 2/76

(54) **VERFAHREN ZUR STEUERUNG EINES PROTHETISCHEN UND/ODER ORTHETISCHEN SYSTEMS UND EIN SOLCHES SYSTEM**
METHOD FOR CONTROLLING A PROSTHETIC AND/OR ORTHOTIC SYSTEM AND SUCH A SYSTEM
PROCÉDÉ DE COMMANDE D'UN SYSTÈME PROTHÉTIQUE ET/OU ORTHÉTIQUE ET UN TEL SYSTÈME

(30) Priorität: 17.12.2021 DE 102021133616; 29.03.2022 DE 102022107365
(43) Veröffentlichungstag der Anmeldung: 23.10.2024
(73) Patentinhaber: Otto Bock Healthcare Products GmbH, 1110 Wien (AT)
(72) Erfinder: PAPPE, Alexander, 1110 Wien (AT); BOHLAND, Andreas, 1110 Wien (AT); WEBER, Martin, 1110 Wien (AT); FINKE, Lars Benjamin, 1110 Wien (AT); SIEGEL, Johannes, 1110 Wien (AT)
(74) Vertreter: Gramm, Lins & Partner Patent- und Rechtsanwälte PartGmbB
(86) Internationale Anmeldenummer: PCT/EP2022/085826
(87) Internationale Veröffentlichungsnummer: WO 2023/111003

(56) Entgegenhaltungen:
- DE-A1- 102019 101 143

## Beschreibung

Die Erfindung betrifft ein Verfahren zur Steuerung eines prothetischen und/oder orthetischen Systems mit einer ersten mechatronischen Komponente und zumindest einer zweiten mechatronischen Komponente, die miteinander verbunden sind, insbesondere lösbar miteinander verbunden sind, wobei jede mechatronische Komponente zumindest einen Aktuator und eine Steuerungseinheit mit einer Steuerungssoftware aufweist, um den Aktuator in Abhängigkeit von Sensorwerten zu aktivieren, modulieren oder zu deaktivieren, wobei Sensordaten von zumindest einem Sensor an zumindest einer der mechatronischen Komponenten zumindest einer der Steuerungseinheiten übermittelt und zur Steuerung des jeweiligen Aktuators durch die Steuerungssoftware verwendet werden und die Steuerungssoftware der mechatronischen Komponenten miteinander kommuniziert. Die Erfindung betrifft ebenfalls ein prothetisches und/oder orthetisches System zur Durchführung eines solchen Verfahrens.

Orthesen sind orthopädietechnische Hilfsmittel, die an einer existierenden Gliedmaße angelegt werden und die Bewegungen führen, einschränken oder unterstützen. Zwischen gelenkig miteinander verbundenen Komponenten können Aktuatoren und/oder Widerstandseinrichtungen, die über einen Aktuator verstellt oder eingestellt werden können, angeordnet sein. Die Verstellung kann auf Basis von Sensordaten, die einer Datenverarbeitungseinrichtung übermittelt werden, erfolgen. Unter Orthesen werden im Rahmen dieser Anmeldung auch Exoskelette verstanden, die an dem Körper eines Patienten angelegt werden und eine äußere Stützstruktur bilden, insbesondere um die Bewegungen eines Nutzers zu führen und zu beeinflussen, z.B. durch Antriebe zu unterstützen oder über Widerstandseinrichtungen zu bremsen. Orthesen und Exoskelette als deren Spezialfälle und motorisch angetriebene Prothesen oder Prothesensysteme können neben der Unterstützung der täglichen Verrichtungen auch zu Trainingszwecken oder zu therapeutischen Zwecken verwendet und eingesetzt werden.

Prothesen ersetzen nicht oder nicht mehr vorhandene Gliedmaßen. Die einfachsten Prothesenkomponenten haben eine rein kosmetische Funktion oder vervollständigen eine Gliedmaße, beispielsweise indem ein distales Fingerglied ersetzt wird. Im Laufe der Zeit wurden die Prothesen komplexer, mehrere Prothesenkomponenten wurden aneinander angeordnet und befestigt und beispielsweise über Gelenke miteinander verbunden. Komplexe mechanische Antriebsvorrichtungen wurden entwickelt, um beispielsweise Prothesenhände oder Prothesenfüße zu bewegen. Hydraulische oder andere Dämpfereinrichtungen oder Widerstandseinrichtungen werden an Gelenken angeordnet, um das Verhalten von Prothesenkomponenten und Prothesensystemen zu modifizieren, um einen möglichst natürlichen Bewegungsablauf zu ermöglichen. Zur Unterstützung von Bewegungen werden Antriebe in Prothesenkomponenten integriert, sodass aktive Prothesen entstanden sind. Weiterhin werden Sensoren an Prothesenkomponenten oder einem Prothesennutzer angeordnet, um das gegenwärtige Bewegungsverhalten oder die gegenwärtigen Positionen oder Stellungen von Prothesenkomponenten zueinander zu erfassen und das zukünftige Bewegungsverhalten abzuschätzen und um Einstellungen an Widerstandseinrichtungen und/oder Antrieben zu verändern. Dadurch sind hochkomplexe Prothesensysteme mit mehreren, aneinander angeordneten Prothesenkomponenten entstanden, die eine Vielzahl von mechanischen, elektrischen und mechatronischen Komponenten aufweisen.

Ein Prothesensystem der unteren Extremität kann insbesondere einen Oberschenkelschaft aufweisen, an dessen distalem Ende ein Prothesenkniegelenk, ein Prothesenunterschenkel und ein Prothesenfuß befestigt sind. Ein solches Prothesensystem weist beispielsweise zwei oder mehr Gelenke auf, die jeweils mit Widerstandseinrichtungen und/oder Antrieben bzw. Aktuatoren versehen sein können.

Aus der EP 2 816 979 B1 ist ein Verfahren zur Steuerung eines künstlichen Orthesenkniegelenkes oder Prothesenkniegelenkes bekannt, bei dem auf der Grundlage der Erfassung eines Absolutwinkels einer Unterschenkelkomponente der Beugewiderstand verändert wird. Der ermittelte Absolutwinkel der Unterschenkelkomponente wird mit einem Schwellwert verglichen, wird der Schwellenwert erreicht oder überschritten, wird der Beugewiderstand verändert.

Aus der EP 2 790 614 B1 ist eine Beinprothese mit einem Oberschenkelteil, einem Unterschenkelteil und einem Fußteil bekannt, die aneinander festgelegt sind. Ein Kniegelenk verbindet das Oberschenkelteil mit dem Schienbeinteil und weist eine dynamisch einstellbare Kniebeugungssteuerungsvorrichtung zur Dämpfung der Kniebeugung auf. Zwischen dem Fußabschnitt und dem Schienbeinabschnitt ist ein Knöchelgelenk angeordnet. Eine Vielzahl von Sensoren ist mit einem elektronischen Steuerungssystem und der Kniebeugungssteuerungsvorrichtung verbunden, um den jeweiligen Widerstand in den Gelenken dynamisch und automatisch zu verändern.

Aus der US 8 057 550 B2 ist eine Prothese oder Orthese der unteren Extremität mit mehreren mechatronischen Vorrichtungen bekannt, die miteinander kommunizieren. Beispiele für mechatronische Vorrichtungen sind ein Prothesenkniegelenk zwischen einem Oberschenkelteil und einem Unterschenkelteil sowie ein Prothesenknöchelgelenk mit einem Unterschenkelteil und einem Prothesenfuß. In einem solchen Prothesensystem oder Orthesensystem kann eine Hauptsteuerungsvorrichtung angeordnet sein, die die Steuerung des Gesamtsystems übernimmt. Die Hauptsteuereinrichtung kann vollständig oder teilweise eine untergeordnete Vorrichtung steuern.

Prothesensysteme oder Orthesensysteme werden vielfach modular aufgebaut und hinsichtlich ihrer Anforderungen an den jeweiligen Patienten angepasst. Dabei ist das Zusammenspiel der jeweiligen mechatronischen Komponenten oder mechatronischen Vorrichtungen als Subsysteme des Gesamtsystems von besonderer Bedeutung. Mechatronischen Komponenten in einem Subsystem werden auf der Grundlage unterschiedlicher Sensorwerte von der jeweiligen Steuerungssoftware separat angesteuert. Dies kann zu Problemen bei der Benutzung des Orthesensystems bzw. des Prothesensystems führen.

Die DE 10 2019 101 143 A1 betrifft ein Verfahren zur Steuerung einer orthetischen oder prothetischen Einrichtung mit einer Gelenkeinrichtung mit einer proximalen Komponente und einer distalen Komponente. Zwischen der proximalen Komponente und der distalen Komponente ist ein verstellbarer Aktuator angeordnet. Eine Erfassungseinrichtung erfasst Muskelkontraktionen, insbesondere Muskelkokontraktionen, des Anwenders. Eine Steuerungseinrichtung ist mit der Erfassungseinrichtung und dem Aktuator gekoppelt und verarbeitet Signale von der Erfassungseinrichtung und verstellt den Aktuator in Abhängigkeit von den Signalen.

Aufgabe der vorliegenden Erfindung ist es, ein orthetisches und/oder prothetisches System sowie ein Verfahren zu dessen Steuerung bereitzustellen, mit denen eine vereinfachte Anpassung an unterschiedliche Nutzungssituationen erfolgen kann.

Erfindungsgemäß wird diese Aufgabe durch ein Verfahren mit den Merkmalen des Hauptanspruches und ein prothetisches und/oder orthetisches System mit den Merkmalen des nebengeordneten Anspruchs gelöst. Vorteilhafte Ausgestaltungen und Weiterbildungen der Erfindung sind in den Unteransprüchen, der Beschreibung sowie den Figuren näher erläutert.

Das Verfahren zur Steuerung eines prothetischen und/oder orthetischen Systems mit einer ersten mechatronischen Komponente und zumindest einer zweiten mechatronischen Komponente, die miteinander verbunden sind, wobei jede mechatronische Komponente zumindest einen Aktuator und eine Steuerungseinheit mit einer Steuerungssoftware aufweist, um den Aktuator in Abhängigkeit von Sensorwerten oder Sensordaten zu aktivieren, modulieren oder zu deaktivieren, wobei Sensordaten von zumindest einem Sensor an zumindest einer der mechatronischen Komponenten zumindest einer der Steuerungseinheiten übermittelt und zur Steuerung des jeweiligen Aktuators durch die Steuerungssoftware verwendet werden und die Steuerungssoftware der mechatronischen Komponenten miteinander kommuniziert, sieht vor, dass anhand der Sensordaten ein Zustand oder ein Bewegungsmuster des prothetischen und/oder orthetischen Systems erkannt und an zumindest eine weitere Steuerungseinheit, insbesondere an alle Steuerungseinheiten übermittelt wird und dass die zumindest eine weitere Steuerungseinheit und die dazugehörige mechatronische Komponente, insbesondere alle Steuerungseinheiten und alle mechatronischen Komponenten in eine Grundkonfiguration gebracht werden. In der Grundkonfiguration befinden sich alle Komponenten oder die jeweiligen Komponenten des orthetischen und/oder prothetischen Systems in einem Ausgangszustand oder einer Ausgangsstellung, von dem aus alle verstellbaren oder einstellbaren Bauteile, Baugruppen und Komponenten oder dergleichen der jeweiligen mechatronischen Komponenten im Verlauf der weiteren Benutzung des Systems gesteuert werden. Sensordaten von zumindest einem Sensor werden an zumindest eine der Steuerungseinheiten der zumindest einen mechatronischen Komponente übermittelt und zur Steuerung des jeweiligen Aktuators durch die Steuerungssoftware verwendet. Wird von einer der mechatronischen Komponenten ein Zustand oder ein Bewegungsmuster eindeutig erkannt oder werden von allen oder mehreren mechatronischen Komponenten ein solcher Zustand oder ein solches Bewegungsmuster eindeutig erkannt oder als solches festgelegt, werden die jeweiligen oder alle Aktuatoren entsprechend aktiviert, deaktiviert oder moduliert, sodass die jeweiligen oder alle mechatronischen Komponenten den gleichen Grundzustand aufweisen, von dem ausgehend jede weitere Aktivität vorgenommen wird. Dabei können die einzelnen Bauteile einer mechatronischen Komponente und/oder die Systemkomponenten des Prothesen- oder Orthesensystems auch verstellt werden, sodass beispielsweise beim Erkennen eines sitzenden Zustandes neben einer Verringerung des Flexionswiderstandes und des Extensionswiderstandes in dem Prothesenkniegelenk auch eine Komponente eines Oberschenkelschaftes verstellt und gleichzeitig der Prothesenfuß in eine Neutralstellung verfahren wird. So kann beispielsweise beim Erkennen eines sitzenden Zustandes neben der Verringerung des Flexionswiderstandes und/oder des Extensionswiderstandes in dem Prothesenkniegelenk der Anpressdruck oder die zirkuläre Kraft, welche vom Schaft auf den Oberschenkel ausgeübt wird, oder lokale Steifigkeiten im Oberschenkelschaft angepasst, vorzugsweise verringert werden. Die Verringerung der Steifigkeit des Prothesenschaftes kann beispielsweise in dem Bereich des Trochanter Major und/oder in dem Bereich der Ramusanlage erfolgen.

Ein anderes Beispiel für ein Zusammenspiel der Aktuatoren oder Komponenten sind die Anpassungen eines prothetischen oder orthetischen Systems an unterschiedliche Gehgeschwindigkeiten. Bei einem schnellen Gehen werden die Dämpfungen in dem Kniegelenk und dem Knöchelgelenk erhöht, ebenso werden der Schaftsitz durch z.B. Anziehen eines Spannsystems gefestigt und die Steifigkeit des Schaftes gegenüber der normalen Gehgeschwindigkeit erhöht, während bei einem langsamen Gehen die Dämpfungen verringert, der Schaftsitz gelockert und die Schaftsteifigkeit verringert werden.

Beim Stehen werden wiederum die Dämpfungen in Knie und Knöchel erhöht und der Schaftsitz gelockert und/oder die Steifigkeit der Schaftwandung reduziert.

Eine andere Aktivität ist das Treppen abwärts Gehen, das beispielsweise durch einen bestimmten Bewegungsablauf und eine bestimmte Belastungsabfolge im Knie durch dessen Steuerungseinheit oder die zentrale Steuerungseinheit erkannt wird. Die Grundkonfiguration des Fußes wird geändert und der Fuß stellt aktiv eine Plantarflexion ein, um einen Auftritt im Vorfußbereich zu erreichen. Gleichzeitig wird die Grundkonfiguration in dem Kniegelenk geändert und der Knieflexionswiderstand wird erhöht, während der Anpressdruck in dem Schaft ebenfalls erhöht wird, um eine ausreichende Sicherheit für den Nutzer zu erreichen.

Der Zustand oder das Bewegungsmuster des prothetischen und/oder orthetischen Systems kann von einer zentralen Steuerungseinheit erkannt werden, die mit den anderen Steuerungseinheiten der jeweiligen mechatronischen Komponenten verbunden ist. Die zentrale Steuerungseinheit hat die Aufgabe und die Berechtigung, den Zustand oder das Bewegungsmuster des prothetischen und/oder orthetischen Systems zu erkennen und diesen Zustand oder das Bewegungsmuster verbindlich für alle anderen Steuerungseinheiten und mechatronischen Komponenten vorzugeben. Alternativ ist die zentrale Steuerungseinheit Teil einer Steuerungseinheit einer mechatronischen Komponente, die als führende mechatronische Komponente bestimmt wird, beispielsweise für den Fall eines orthetischen und/oder prothetischen Systems einer unteren Gliedmaße ein künstliches Kniegelenk.

In einer Ausgestaltung wird ausgehend von der Grundkonfiguration für denjenigen Zustand oder das jeweilige Bewegungsmuster die weitere Steuerung dezentral über die den mechatronischen Komponenten zugeordneten Steuerungseinheiten durchgeführt. Die Erkennung und Bestimmung der Grundkonfiguration auf Grundlage des erkannten Zustandes bzw. des erkannten Bewegungsmusters vereinheitlicht den Steuerungsrahmen, innerhalb dessen sich das prothetische und/oder orthetische System befindet. Das Gesamtsystem ist damit in einen gemeinsamen, einheitlichen Anfangszustand gebracht worden, von dem aus die einzelnen Untersysteme bis zum Erkennen eines anderen Zustandes bzw. eines anderen Bewegungsmusters auf der Grundlage von Sensordaten gesteuert werden. Die Steuerung der einzelnen mechatronischen Komponenten mit den jeweils zugeordneten Steuerungseinheiten erfolgt dabei nicht nur auf der Grundlage der Sensordaten, die dem jeweiligen Steuerungseinheit zugeordnet sind, vielmehr besteht die Möglichkeit, dass Sensordaten einer ersten mechatronischen Komponente die Grundlage für die Verstellung eines Aktuators in einer anderen mechatronischen Komponente bilden oder zumindest dazu mit berücksichtigt werden.

In einer Ausgestaltung erfolgt die weitere Steuerung alle Aktuatoren der mechatronischen Komponenten über eine zentrale Steuerungseinheit, nachdem ein vorbestimmte Zustand oder ein vorbestimmtes Bewegungsmuster erkannt worden ist. Diese Steuerung über die zentrale Steuerungseinheit erfolgt so lange, bis ein anderer Zustand oder ein anderes Bewegungsmuster erkannt wird. Die zentrale Steuerungseinheit steuert dann alle mechatronischen Komponenten oder zumindest die betroffenen Komponenten sowohl hinsichtlich des Zustandes als auch hinsichtlich der Einstellung in dem jeweiligen Zustand oder der jeweiligen Situation. Eine solche zentrale Steuerung kann für vorgegebene, bestimmte Zustände und/oder Bewegungsmuster eingerichtet sein, für andere Zustände und/oder Bewegungsmuster erfolgt beispielsweise eine dezentrale Steuerung. Alternativ dazu dient die zentrale Steuerungseinheit nur zur Erkennung des Zustandes bzw. des Bewegungsmusters, was an alle anderen Steuerungseinrichtungen weitergegeben und verbindlich vorgegeben wird. Die jeweilige Steuerung der Aktuatoren der mechatronischen Komponente erfolgt dann von den untergeordneten bzw. lokalen Steuerungseinrichtungen.

Sofern eine zentrale Steuerungseinheit vorhanden ist, ist diese vorteilhafterweise mit mehreren, insbesondere allen Sensoren verbunden. Die Sensoren können drahtlos oder per Kabel mit der Steuerungseinheit bzw. mit den jeweiligen Steuerungseinheiten verbunden sein, sodass drahtlos oder per Kabel die Sensordaten zwischen den mechatronischen Komponenten und insbesondere den jeweiligen Steuerungseinheiten der mechatronischen Komponenten ausgetauscht werden.

In einer Ausgestaltung wird auf der Grundlage der Sensordaten eine Steuerungseinheit einer der mechatronischen Komponenten als zentrale Steuerungseinheit bestimmt. Die Bestimmung erfolgt beispielsweise auf der Grundlage der Art der Sensordaten, der Menge der Sensordaten und/oder der Qualität der Sensordaten. Liegen beispielsweise überwiegend Daten hinsichtlich der Bewegung eines Oberschenkelteils relativ zu einem Unterschenkelteil vor, während keinerlei Belastungsdaten eines Prothesenfußes oder einer Fußplatte eine Orthese vorhanden sind, ist es vorgesehen, dass die Steuerungseinheit für das künstliche Kniegelenk als zentrale Steuerungseinheit bestimmt wird. Die Bestimmung erfolgt durch Kommunikation aller Steuerungseinheiten mit einer entsprechenden Abfrage. Dazu sind sogenannte State Machines in den jeweiligen Steuerungseinheiten abgelegt, auf deren Grundlage beispielsweise erkannt wird, ob ein Nutzer der Orthese und/oder Prothese sitzt, liegt, steht oder geht bzw. wie der jeweilige Zustand beschaffen ist oder wie die jeweilige Bewegung ausgeführt wird.

In einer Ausgestaltung sind alle mechatronischen Komponenten aus autonom funktionierenden Komponenten ausgebildet, die lösbar aneinander befestigt werden können. Die autonom funktionierenden Komponenten weisen zumindest einen Sensor, einen Aktuator, einen Energiespeicher eine Schnittstelle und eine Steuerungseinheit auf, wobei die Steuerungseinheit eine Steuerungssoftware aufweist, um den Aktuator in Abhängigkeit von den Sensorwerten des zumindest einen Sensors zu aktivieren, zu modulieren oder zu deaktivieren.

In einer Ausgestaltung ist eine mechatronische Komponente ein orthopädietechnischer Schaft, insbesondere ein Prothesenschaft, der eine Schaftwand aufweist, deren Steifigkeit zumindest bereichsweise reversibel über zumindest einen Aktuator in Abhängigkeit von den Sensordaten, insbesondere während der Benutzung des Schaftes in Echtzeit und/oder in Abhängigkeit von der Bewegungssituation, verändert wird. Der orthopädietechnische Schaft zur Anlage an und zur Aufnahme von einem Gliedmaßenstumpf oder einer Gliedmaße mit zumindest einer Schaftwand weist insbesondere eine proximale Einstiegsöffnung, ein distales Ende und zumindest eine Befestigungseinrichtung, die zur Festlegung zumindest einer distalen orthopädietechnischen Komponente an dem Schaft angeordnet ist, auf. An oder in der Schaftwand ist zumindest ein Versteifungselement angeordnet, dem z.B. eine Verstelleinrichtung mit einem Aktuator zugeordnet ist.

Über das Versteifungselement, insbesondere über eine Aktivierung, Deaktivierung oder Modulierung des Aktuators, ist die Steifigkeit der Schaftwand zumindest bereichsweise veränderbar, insbesondere reversibel veränderbar. Mit einem solchen Schaft ist es möglich, dass bestimmte Flächen oder Bereiche der Schaftwand nur dann eine notwendige Steifigkeit oder Rigidität aufweisen, wenn dies notwendig ist. Insbesondere kann bei unterschiedlichen Nutzungssituationen die Steifigkeit bereichsweise oder sektorweise angepasst werden, um entweder eine präzise Führung und eine stabile Abstützung der Gliedmaße oder des Gliedmaßenstumpfes zu gewährleisten oder um eine ausreichende Nachgiebigkeit bereitzustellen, damit der Schaft angenehm zu tragen ist. Die situationsbedingte oder frei gewählte, reversible Veränderbarkeit der Steifigkeit in bestimmten Bereichen hat zur Folge, dass der Schaft als solches in seinen mechanischen Eigenschaften eingestellt werden kann und dass durch die situationsbedingte Einstellbarkeit eine optimale Funktionalität bereitgestellt wird.

Das Versteifungselement wirkt in Kombination mit der Schaftwand und dient dazu, die Steifigkeit in den entsprechenden Bereichen zu verändern. Das Versteifungselement ist insbesondere eine Zusatzkomponente an einem Schaft, die daran befestigt, angeordnet, ausgebildet oder integriert ist.

Eine Variante sieht vor, dass dem Versteifungselement ein motorischer Antrieb zugeordnet ist, z.B. über ein Verstellelement, das zwischen dem Antrieb oder der Verstelleinrichtung und dem Versteifungselement angeordnet ist. Über den Antrieb oder die Verstelleinrichtung ist es möglich, das Verstellelement und/oder das Versteifungselement zu verdrehen, zu verschieben, zu verbiegen oder auf eine andere Art und Weise zu verlagern oder zu manipulieren, um die gewünschte Veränderung der Steifigkeit in dem jeweiligen Bereich der Schaftwand zu erreichen. Als motorischer Antrieb wird auch ein linearer Aktuator angesehen, ebenso schaltbare Magneteinrichtungen, die eine Verlagerung bewirken. Die Veränderung der Steifigkeit erfolgt in einer Ausgestaltung durch eine Verlagerung eines Verstellelementes, eine Verstellung, Verlagerung oder Veränderung eines Versteifungselementes, das an oder in der Schaftwand angeordnet ist und/oder durch Aktivierung oder Deaktivierung der Verstelleinrichtung. Die Veränderung des Versteifungselementes erfolgt beispielsweise durch eine Verschiebung, eine Stauchung, eine Veränderung eines Anstellwinkels eines Abstützelementes oder das Einführen oder Entfernen von Material in Zwischenräumen zwischen Abschnitten, die durch das Versteifungselement ausgebildet werden.

In einer Weiterbildung weist die Schaftwand Sektoren auf, die um den Umfang verteilt sind, die unabhängig voneinander hinsichtlich ihrer Steifigkeit verändert werden. Die Veränderung der Steifigkeit erfolgt in einer Ausgestaltung während der Benutzung des orthopädietechnischen Schaftes automatisch, vorteilhafterweise in Echtzeit. Dabei werden die Daten von Sensoren aufgenommen, einer Auswerte- und Steuerungseinrichtung übermittelt und auf der Grundlage dieser Sensordaten und der Auswertung wird die Verstelleinrichtung aktiviert, deaktiviert oder moduliert, um den jeweils gewünschten oder erforderlichen Grad an Steifigkeit einzustellen. Dadurch ist es möglich, an die jeweilige Situation angepasste Steifigkeiten einzustellen, insbesondere aufgeteilt nach Sektoren, für die in dem proximalen Randbereich eine Anpassung an die Notwendigkeiten oder Vorlieben des jeweiligen Nutzers erfolgt. Die Sensordaten können von Sensoren stammen, die an dem Schaft angeordnet und/oder an einer Komponente angeordnet oder dieser zugeordnet sind, die an dem Schaft befestigt ist. Ist beispielsweise eine Widerstandseinrichtung Teil der orthopädietechnischen Einrichtung, an der der orthopädietechnische Schaft angeordnet ist, können die Sensoren, die für die Steuerung der Widerstandseinrichtung verwendet werden, auch zur Erkennung von Belastungssituationen eingesetzt werden. Auf der Grundlage der jeweiligen Belastungssituationen kann dann nicht nur der Widerstand eingestellt, sondern auch die Steifigkeit in dem jeweiligen Sektor des Schaftes verändert werden.

Ein prothetisches und/oder orthetisches System zur Durchführung des oben beschriebenen Verfahrens mit einer ersten mechatronischen Komponente und zumindest einer zweiten mechatronischen Komponente, die miteinander verbunden, insbesondere lösbar miteinander verbunden sind, wobei jede mechatronische Komponente zumindest einen Aktuator und eine Steuerungseinheit mit einer Steuerungssoftware aufweist, um den Aktuator in Abhängigkeit von Sensorwerten zu aktivieren, modulieren oder zu deaktivieren, wobei Sensordaten von zumindest einem Sensor an zumindest einer der Komponenten einer der Steuerungseinheiten übermittelt und zur Steuerung eines oder des jeweiligen Aktuators durch die Steuerungssoftware verwendet werden und die Steuerungssoftware der Komponenten miteinander kommuniziert, sieht vor, dass die Steuerungssoftware eingerichtet ist, anhand der Sensordaten einen Zustand oder ein Bewegungsmuster des prothetischen und/oder orthetischen Systems zu erkennen und an alle anderen Steuerungseinheiten zu übermitteln und damit zumindest eine weitere Steuerungseinheit, insbesondere alle Steuerungseinheiten und die dazugehörigen Komponenten in eine Grundkonfiguration zu bringen. Sensordaten von zumindest einem Sensor werden an zumindest eine Steuerungseinheit der zumindest einen Komponente übermittelt und zur Steuerung eines oder des jeweiligen Aktuators durch die Steuerungssoftware verwendet. Die Steuerungssoftware zumindest einer der mechatronischen Komponenten ist dazu eingerichtet, den Zustand oder ein Bewegungsmuster einer weiteren mechatronischen Komponente, insbesondere des gesamten prothetischen und/oder orthetischen Systems zu erkennen, alternativ weist die Steuerungssoftware aller mechatronischen Komponenten diese Funktionalität auf.

Die mechatronischen Komponenten weisen in einer Ausgestaltung Befestigungseinrichtungen zum lösbaren Festlegen aneinander auf, sodass unterschiedliche mechatronische Komponenten ähnlicher Art ausgewechselt und mit anderen proximalen oder distalen mechatronischen Komponenten kombiniert werden können. Die Befestigungseinrichtungen sind beispielsweise Pyramidenadapter, Spanneinrichtungen, Verschraubungen, Clipselemente, Bajonettverschlüsse, Steckverbindungen oder andere Ausgestaltungen, mit denen zwei Elemente dauerhaft und lösbar aneinander festgelegt werden können. So können beispielsweise unterschiedliche künstliche Kniegelenke mit unterschiedlichen Aktuatoren und/oder Widerstandseinrichtungen mit Aktuatoren zu deren Verstellung mit einem Oberschenkelschaft und einem Unterschenkelrohr kombiniert werden.

Jede mechatronische Komponente weist eine autonome Funktionalität auf und weist insbesondere eine eigene Schnittstelle zum Transport von Energie und/oder Daten auf.

In einer Ausgestaltung ist eine mechatronische Komponente als orthopädietechnischer Schaft ausgebildet und weist zumindest eine Schaftwand auf, an der zumindest ein Versteifungselement angeordnet ist, über das die Steifigkeit der Schaftwand zumindest bereichsweise reversibel veränderbar ist.

In einer Ausgestaltung ist der orthopädietechnische Schaft ein Prothesenschaft, der eine Schaftwand aufweist, deren Steifigkeit zumindest bereichsweise reversibel über zumindest einen Aktuator in Abhängigkeit von den Sensordaten, insbesondere während der Benutzung des Schaftes in Echtzeit, verändert wird. Der orthopädietechnische Schaft zur Anlage an und zur Aufnahme von einem Gliedmaßenstumpf oder einer Gliedmaße mit zumindest einer Schaftwand weist insbesondere eine proximale Einstiegsöffnung, ein distales Ende und zumindest eine Befestigungseinrichtung, die zur Festlegung zumindest einer distalen orthopädietechnischen Komponente an dem Schaft angeordnet ist, auf. An oder in der Schaftwand ist zumindest ein Versteifungselement angeordnet, dem z.B. eine Verstelleinrichtung mit einem Aktuator zugeordnet ist. Über das Versteifungselement, insbesondere über eine Aktivierung, Deaktivierung oder Modulierung des Aktuators, ist die Steifigkeit der Schaftwand zumindest bereichsweise veränderbar, insbesondere reversibel veränderbar ist. Mit einem solchen Schaft ist es möglich, dass bestimmte Flächen oder Bereiche der Schaftwand nur dann eine notwendige Steifigkeit oder Rigidität aufweisen, wenn dies notwendig ist.

Die Schaftwand weist in einer Ausführungsform Sektoren auf, die über den Schaftumfang verteilt sind und deren Steifigkeit unabhängig voneinander reversibel veränderbar ist. Die Sektoren können unterschiedliche Materialien, unterschiedliche Materialstärken, unterschiedlich breite Ausnehmungen und/oder unterschiedliche Dichten an Ausnehmungen aufweisen. Werden beispielsweise die Versteifungselemente vollständig deaktiviert oder auf den jeweils minimalen Widerstand eingestellt, können sich unterschiedliche Grundsteifigkeiten oder Grundwiderstände gegen eine radiale Verlagerung des Schaftes aufgrund der grundsätzlichen Konstruktion in dem proximalen Randbereich ergeben. Die unterschiedlichen Grundsteifigkeiten können durch unterschiedliche Materialien in den unterschiedlichen Sektoren bereitgestellt werden. Sofern gleiche Materialien verwendet werden, beispielsweise ein Kunststoff oder ein faserverstärkter Kunststoff, können unterschiedliche Materialstärken zu unterschiedlichen Steifigkeiten in den jeweiligen Sektoren führen. Ergänzend oder alternativ zu den beiden vorgenannten Maßnahmen können Ausnehmungen innerhalb der Sektoren vorgesehen sein, die die Steifigkeit des jeweiligen Abschnittes der Schaftwand beeinflussen. Eine Möglichkeit zur Ausgestaltung der Ausnehmungen sind Schlitze, die in proximaldistal-Richtung eingebracht sind. Die Schlitze können bis zum oberen Abschluss des Prothesenschaftes reichen und erstrecken sich in distaler Richtung gegebenenfalls bis zu dem Ende des proximalen Randbereiches. Die Breite der Ausnehmungen oder Schlitze kann zwischen den Sektoren oder auch innerhalb eines Sektors variieren. Ebenso kann die Dichte der Ausnehmungen der einzelnen Sektoren verschieden sein. Je höher die Anzahl der Ausnehmungen auf einer normierten Umfangslänge ist, desto geringer ist die Steifigkeit, je höher die Länge der Ausnehmungen ist, desto geringer ist die Steifigkeit und je größer die Breite der Ausnehmungen ist, desto geringer ist die Steifigkeit in dem jeweiligen Segment.

In einer Ausgestaltung sind den einzelnen Sektoren verlagerbare Schaftwandsegmente als Versteifungselemente zugeordnet, die einzeln oder auch in Kombination miteinander aktuiert oder verlagert werden, um unterschiedliche Steifigkeiten in dem proximalen Randbereich bereitzustellen.

In einer Ausgestaltung ist in der Schaftwand zumindest ein flexibler Bereich ausgebildet, der mit einem Versteifungselement gekoppelt ist. Der flexible Bereich lässt beispielsweise immer eine Verlagerung des Materials innerhalb des flexiblen Bereiches radial nach innen in Richtung auf den Stumpf oder die Gliedmaße zu. Ist das Versteifungselement entspannt oder nicht aktiviert, lässt sich das Material des flexiblen Bereiches auch in die entgegengesetzte Richtung bewegen, sodass ein vergrößertes Spiel oder eine vergrößerte Beweglichkeit der Gliedmaße oder des Stumpfes in diesem Sektor erreicht werden kann. Das Versteifungselement ist beispielsweise ein Zugmittel wie ein Gurt oder ein Seil, das gespannt wird, sodass eine Verlagerung des Materials radial nach außen, beispielsweise eines Schaumstoffes oder eines Textils, nicht oder nur in einem beschränkten Maß möglich ist. Eine nicht vorhandene oder verringerte Nachgiebigkeit radial nach außen ist bei hohen Belastungen, beispielsweise bei der schnellen Ausführung von Tätigkeiten, vorteilhaft. Umgekehrt ist eine Entspannung des Zugmittels beispielsweise bei einer Inaktivität vorteilhaft, wenn keine Belastungen aufgenommen werden müssen und nur sichergestellt sein muss, dass sich der Schaft nicht von der Gliedmaße oder dem Stumpf des Nutzers löst.

In einer Ausgestaltung ist eine erste mechatronische Komponente ein künstliches Kniegelenk mit einem Oberteil und einem Unterteil, die schwenkbar aneinander gelagert sind und an denen ein Aktuator angeordnet ist. Der Aktuator ist beispielsweise als Antrieb, Widerstandseinrichtung oder eine Verstelleinrichtung zur Beeinflussung eines Widerstandes ausgebildet. Die zweite mechatronische Komponente kann als künstliches Hüftgelenk, als Oberschenkelschaft, als eine Befestigungseinrichtung und/oder ein künstliches Knöchelgelenk ausgebildet sein und ebenfalls einen Aktuator und eine Steuerungseinrichtung aufweisen. Bevorzugt ist die erste mechatronische Komponente in Gestalt eines künstlichen Kniegelenkes als Master-Komponente und die zumindest eine zweite mechatronische Komponente als sogenannte Slave Komponente ausgebildet, die in der Steuerungshierarchie der Masterkomponente untergeordnet ist.

Insbesondere bei komplexen prothetischen und/oder orthetischen Systemen ist die Kopplung der einzelnen mechatronischen Komponenten schwierig. Eine Kombination aus orthetischen Systemen und prothetischen System ist beispielsweise gegeben, wenn eine Hüftorthese vorhanden ist, die an einem Oberschenkelstumpf festgelegt ist, an dem wiederum ein Prothesenkniegelenk über einen Oberschenkelschaft befestigt ist. Das Prothesenkniegelenk ist mit einem Unterschenkelrohr und einem gelenkig daran befestigten Prothesenfuß verbunden. Zwischen den einzelnen Gelenken sind Aktuatoren angeordnet, beispielsweise passive Widerstandseinrichtungen wie Hydraulikdämpfer oder aktive Antriebe wie Elektromotoren, die Pumpen oder Getriebe antreiben oder als Generatoren Relativbewegungen zwischen einzelnen Komponenten abbremsen. Die Aktuatoren können auch Ventile in passiven Widerstandseinrichtungen verstellen, Strömungswiderstände über Veränderung von Magnetfeldern verändern oder auf andere Art und Weisen eine Relativbewegung zwischen beweglichen Bestandteilen einer oder mehrerer mechatronischen Komponenten beeinflussen. Die mechatronischen Komponenten werden aufgrund der individuellen Versorgung des Patienten an den jeweiligen Einsatzzweck und den jeweiligen Patienten angepasst, ausgewählt und zusammengestellt. Die Befestigungseinrichtungen zur Festlegung der mechatronischen Komponenten aneinander sind häufig so ausgebildet, dass auch mechatronische Komponenten unterschiedlicher Hersteller miteinander kombinierbar sind. Ein verstellbarer Oberschenkelschaft kann mit nahezu jedem beliebigen Prothesenkniegelenk gekoppelt werden, gleiches gilt für ein Unterschenkelrohr mit einem Prothesenknöchelgelenk.

Zur Steuerung des Gesamtsystems aus mehreren mechatronischen Komponenten ist es vorteilhaft, einen einmal erkannten Zustand, beispielsweise über eines State Machine, allen weiteren Komponenten verbindlich mitzuteilen und diese Komponenten in eine Grundkonfiguration zu bringen, von der aus alle weiteren Aktivitäten hinsichtlich des Gesamtsystems auf der Grundlage der Sensordaten durchgeführt werden. Damit wird verhindert, dass beispielsweise ein verstellbarer Oberschenkelschaft auf der Grundlage seiner Sensordaten einen Anpressdruck an den Oberschenkelstumpf verringert, da als Zustand das Sitzen angenommen wird, während das Prothesenkniegelenk auf der Grundlage dessen Sensordaten einen motorischen Antrieb aktiviert, da auf Grundlage dessen Sensordaten ein Bewegungszustand des schnellen Gehens mit einer aktiven Unterstützung vorgesehen wäre. Eine solche Kombination wäre potentiell gefährlich. Um das Zusammenspiel aller Komponenten, insbesondere aller mechatronischen Komponenten zu verbessern, wird daher eine verbindliche Vorgabe über den jeweiligen Zustand an alle Steuerungseinheiten aller mechatronischen Komponenten übermittelt. Die nach der Einstellung der Grundkonfiguration durchgeführte Steuerung kann entweder zentral über eine zentrale Steuerungseinheit oder dezentral von den jeweiligen Steuerungseinheiten unter der Berücksichtigung des einheitlich festgestellten Zustandes oder Bewegungsmusters erfolgen.

Bei einer Ausgestaltung einer mechatronischen Komponente als Oberschenkelschaft mit zumindest einer Schaftwand ist an dieser zumindest ein Versteifungselement angeordnet, über das die Steifigkeit der Schaftwand zumindest bereichsweise reversibel veränderbar ist.

Nachfolgend wird ein Ausführungsbeispiel der Erfindung anhand der Figuren näher erläutert. Gleiche Bezugszeichen bezeichnen gleiche Komponenten. Es zeigen:
Figur 1 - eine schematische Darstellung eines prothetischen Systems;
Figuren 2 bis 6 - Integrationsstufen der Steuerung;
Figur 7 - einen Prothesenschaft mit Versteifungselement;
Figur 8 - eine perspektivische Ansicht eines Prothesenschaftes mit Sektoren; sowie
Figur 9 - eine Steuerung der Verstellung von Steifigkeiten.

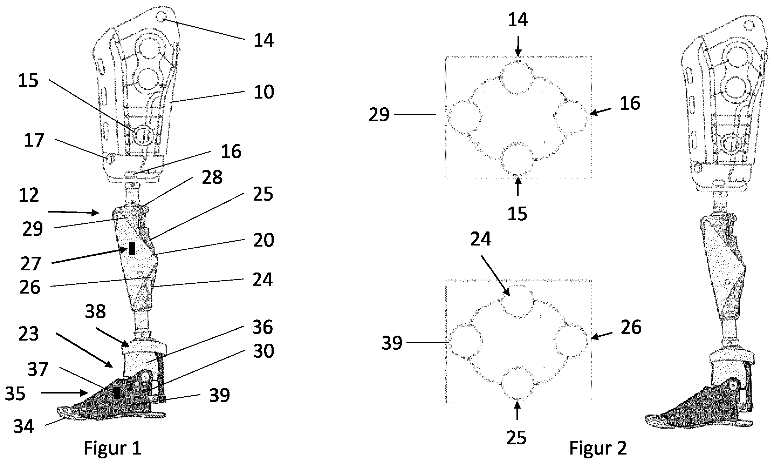

In der Figur 1 ist ein orthopädietechnisches System in Gestalt einer Prothese der unteren Extremität dargestellt. Das orthopädietechnische System ist ein Prothesenbein mit einem Oberschenkelschaft als erste mechatronische Komponente 10, einem Prothesenkniegelenk 12 mit einem Unterschenkelteil als zweite mechatronische Komponente 20 sowie einem Prothesenfuß mit einem Knöchelgelenk als dritte mechatronische Komponente 30. Die erste mechatronische Komponente 10 weist einen Aktuator 15 auf. Der Aktuator 15 ist insbesondere als elektromotorischer Antrieb ausgebildet, der in dem Ausführungsbeispiel dazu dient, verlagerbare Komponenten oder Bauteile der ersten mechatronischen Komponente 10 zueinander zu verlagern. Dazu wird der Aktuator 15 in die eine oder andere Richtung aktiviert, beispielsweise in einer entsprechende Drehrichtung verdreht, sodass ein Kabel, eine Schnur oder eine andere Kraftübertragungseinrichtung eines Zugsystems gespannt bzw. entspannt wird. Durch die Entspannung und die Spannung werden die einzelnen Bauteile des Prothesenschaftes zueinander verlagert, um sich an die Gliedmaße anzupassen. Der Aktuator 15 der ersten mechatronischen Komponente 10 wird über eine Steuerungseinheit 16 aktiviert, deaktiviert oder in der jeweiligen Aktivität moduliert. Die Steuerungseinheit 16 weist die notwendigen Komponenten wie Speichereinrichtungen, Prozessoren, Schnittstellen, Energieversorgungseinrichtungen und dergleichen auf oder ist mit diesen Komponenten verbunden, um auf der Grundlage von Sensorwerten eine entsprechende Aktion auszuführen. An der ersten mechatronischen Komponente 10 ist zumindest ein Sensor 14 oder eine Sensoreinrichtung angeordnet, über den oder die beispielsweise Winkelstellungen, Raumlagen, Drücke, Temperaturen, Geschwindigkeiten, Beschleunigungen und/oder andere Zustandsgrößen oder Bewegungsgrößen erfasst werden. Wenn unterschiedliche Zustandsgrößen oder Parameter erfasst werden, die nicht durch einen einzelnen Sensor erfasst oder aus Sensordaten eines einzelnen Sensors abgeleitet werden können, beispielsweise durch Ableitung nach der Zeit, sind statt eines einzelnen Sensors mehrere Sensoren zu einer Sensoreinrichtung zusammengefasst. Die Sensoreinrichtung gilt in dem Zusammenhang der Anmeldung als Sensor. Der Sensor 14 und damit auch eine ggf. vorhandene Sensoreinrichtung ist mit der Steuerungseinheit 16 gekoppelt. Ebenso ist eine Schnittstelle 17 an der ersten mechatronischen Komponente 10 angeordnet oder ausgebildet und mit der Steuerungseinheit 16 gekoppelt, insbesondere um Energie und/oder Daten zu transportieren.

An dem distalen Ende der ersten mechatronischen Komponente 10 ist das Prothesenkniegelenk 12 angeordnet, das ein Oberteil 28 und ein Unterteil 29 aufweist, die schwenkbar um eine Schwenkachse miteinander verbunden sind. Das Prothesenkniegelenk 12 mit einem Unterschenkelteil weist ebenfalls einen Aktuator 25 auf, sodass die mechanischen Komponenten zusammen mit dem Aktuator 25 die zweite mechatronische Komponente 20 bilden. Der Aktuator 25 kann als Motor oder Antrieb zur Bewirkung einer aktiven Verlagerung von Oberteil 28 und Unterteil 29 zu ausgebildet sein, alternativ ist der Aktuator 25 als Antrieb zur Verstellung von Ventilen oder zur Veränderung von Viskositäten z.B. bei magnetorheologischen Fluiden ausgebildet, um einen Widerstand gegen eine Relativverlagerung von Oberteil und Unterteil des Prothesenkniegelenkes 12 zu verändern. An der zweiten mechatronischen Komponente 20 ist neben dem Aktuator 25 eine separate Steuerungseinheit 26 angeordnet, die mit zumindest einem Sensor 24 gekoppelt ist. Darüber hinaus verfügt die zweite mechatronische Komponente 20 über eine eigene Schnittstelle 27 zum Austausch von Daten und/oder Energie.

An dem distalen Ende der zweiten mechatronischen Komponente 20 ist die dritte mechatronische Komponente 30 in Gestalt eines Prothesenfußes 39 gelenkig gelagert. Die dritte mechatronische Komponente 30 weist ein Knöchelgelenk 23 auf und ist mit dem gelenkig gelagerten Oberteil 38 des Knöchelgelenkes 23 an dem distalen Ende des Unterschenkelteils bzw. an dem distalen Ende der zweiten mechatronischen Komponente 20 festgelegt. Auch die dritte mechatronische Komponente 30 weist einen Aktuator 35 auf, über den beispielsweise die Stellung des Prothesenfußes 39 relativ zu dem Unterschenkelteil oder einer beweglichen Komponente in dem Prothesenfuß 39, beispielsweise einem Zehenteil, verstellt werden kann. Beispielhaft ist ein Sensor in Gestalt eines Drucksensors 34 an dem Sohlenteil des Prothesenfußes 39 angeordnet. Der Sensor 34 oder weitere Sensoren 34 oder eine Sensoreinheit können an unterschiedlichen Stellen an den jeweiligen mechatronischen Komponenten 10, 20, 30 angeordnet sein und unterschiedliche Zustandsgrößen und/oder Bewegungsgrößen ermitteln, insbesondere Winkelstellungen, Beschleunigungen, Raumlagen, Drücke, Kräfte, Momente, Temperaturen und dergleichen. Auch die dritte mechatronische Komponente 30 weist eine Steuerungseinheit 36 auf, über die der zumindest eine Aktuator 35 angesteuert wird, um den jeweiligen Antrieb zu aktivieren, deaktivieren oder einen Sollwert zu verändern.

In dem Ausführungsbeispiel sind alle elektronischen Komponenten 10, 20, 30 miteinander gekoppelt und datentechnisch miteinander verbunden, wobei die jeweilige Steuerungssoftware der Steuerungseinheiten 16, 26, 36 aller mechatronischen Komponenten 10, 20, 30 miteinander kommunizieren. Somit wird ein Datenaustausch insbesondere hinsichtlich der Sensordaten zwischen den einzelnen Steuerungseinheiten 16, 26, 36 gewährleistet.

Alternativ zu einer Ausgestaltung als prothetisches System kann ein entsprechender Aufbau auch als orthetisches System verwirklicht sein. Statt eines Oberschenkelschaftes zum Aufnehmen eines Oberschenkelstumpfes ist bei einer Orthese eine Aufnahmeeinrichtung für einen Oberschenkel vorhanden, beispielsweise eine Schale, eine Schiene oder dergleichen. Statt eines Prothesenkniegelenkes ist zumindest ein künstliches Kniegelenk medial und/oder lateral zu dem natürlichen Kniegelenk angeordnet, um eine Unterschenkelschiene statt eines Unterschenkelteils mit der Oberschenkelschiene zu verbinden. Auch hier kann zwischen dem Oberschenkelteil und dem Unterschenkelteil ein Aktuator angeordnet sein, insbesondere als Antrieb und/oder eine Widerstandseinrichtung. Statt eines Prothesenfußes ist bei einer Orthese ein Aufsatz für einen Fuß gelenkig an der Unterschenkelschiene angeordnet. Auch hier sind Aktuatoren, Sensoren, Steuerungseinrichtung und Schnittstellen vorhanden und entsprechende Steuerungssoftware in den jeweiligen mechatronischen Komponenten abgelegt.

Alternativ zu einer Ausgestaltung einer Orthese oder Prothese der unteren Extremität kann diese auch als eine Orthese oder Prothese der oberen Extremität ausgebildet sein.

In der Figur 2 ist der grundsätzliche Aufbau der einzelnen mechatronischen Komponenten an dem Beispiel zweier mechatronischer Komponenten 10 und 20 dargestellt. Die Sensoren 14, 24 einer jeden mechatronischen Komponenten kommunizieren mit der Steuerungseinheit 16, 26, die wiederum auf den jeweiligen Aktuator 15, 25 einwirkt, die dann auf die einzelnen Komponenten oder Bauteile der mechatronischen Komponente einwirken, beispielweise dem Unterteil 29, was wiederum von den Sensoren 14, 24 erfasst wird. Jede mechatronische Komponente 10, 20 weist eine entsprechende Steuerung auf, mit der beispielsweise das Prothesenkniegelenk 12 mit dem Aktuator 25 entsprechend angesteuert werden kann.

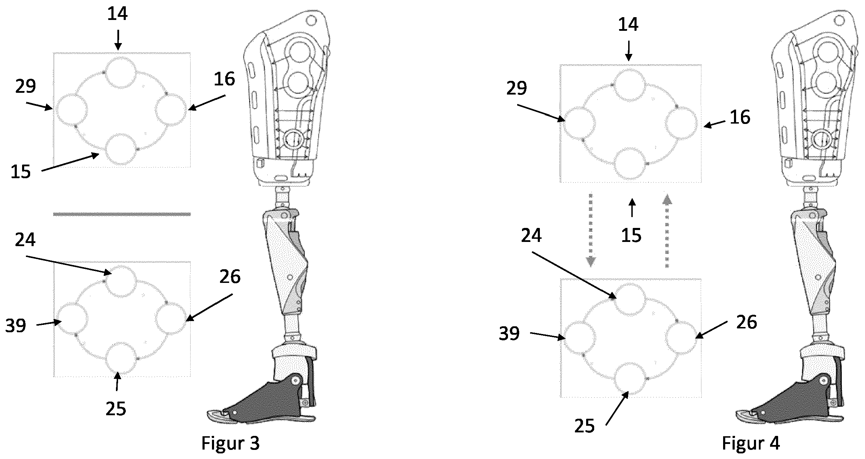

In der Figur 3 ist ein Steuerungsaufbau dargestellt, bei dem jede elektronische Komponente, dargestellt an dem Beispiel einer ersten mechatronischen Komponente 10 in Gestalt eines verstellbaren Prothesenschaftes und einer zweiten mechatronischen Komponente 20 in Gestalt eines Prothesenkniegelenkes mit Unterschenkelteil, separat voneinander gesteuert wird. Eine Kommunikation der Steuerungssoftware findet nicht statt, insbesondere hinsichtlich der Sensorwerte findet keine Kommunikation statt.

In der Figur 4 ist der Modus dargestellt, bei dem die einzelnen Sensorsignale ebenso wie die Signale zum Aktivieren, Deaktivieren oder Modulieren des Aktuators oder der Aktuatoren 15, 25 ausgetauscht werden. Der Austausch findet zwischen beiden mechatronischen Komponenten 10, 20 statt, sodass eine abgestimmte Steuerung des Gesamtsystems erfolgen kann.

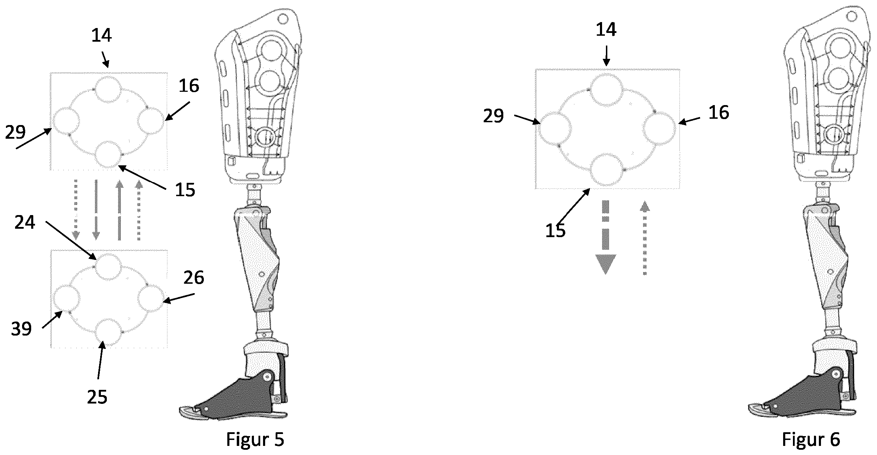

In der Figur 5 ist eine weitere, höhere Integrationsebene der Steuerung dargestellt, bei der neben den Signalen auch der jeweilige Zustand der einzelnen Komponenten 10, 20 übermittelt wird, sodass beispielsweise anhand des Zustandes des Prothesenkniegelenkes 12 erkannt wird, dass der Nutzer der prothetischen Einrichtung sitzt oder geht.

In der Figur 6 wird auf der Grundlage der Kommunikation sowohl der Signale als auch der jeweiligen Zustände der einzelnen mechatronischen Komponenten 10, 20 bestimmt, welches mechatronische System als führendes System oder Master-System anzusehen ist. Dieses Master-System oder diejenige mechatronische Komponente, die als Master angesehen oder festgelegt wird, legt den Zustand oder das Bewegungsmuster verbindlich für alle angeschlossenen mechatronischen Komponenten fest, was dann für die weitere Steuerung maßgeblich ist. Wird beispielsweise über das Prothesenkniegelenk 12 erkannt, dass der Nutzer sitzt, werden alle weiteren elektronischen Komponenten entsprechend darauf eingestellt, insbesondere in eine Grundkonfiguration gebracht, von der aus dann alle weiteren Einstellungen oder Verstellungen vorgenommen werden. So wird im Sitzen beispielsweise der Extensionswiderstand und der Flexionswiderstand in dem Prothesenkniegelenk verringert und der Anpressdruck des Prothesenschaftes an dem Stumpf gelockert, um die Blutzirkulation zu verbessern oder zu optimieren. Gleichzeitig kann der Widerstand hinsichtlich einer Plantarflexion oder Dorsalflexion in dem Prothesenknöchelgelenk 23 verringert werden, um den Prothesenfuß 39 bei leichten Pendelbewegungen des Beines ohne Aufwand flach auf dem Boden aufliegen zu lassen.

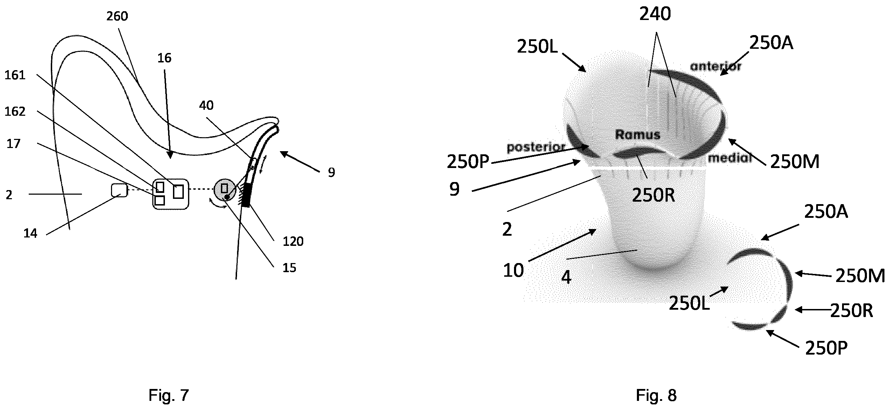

In der Figur 7 ist ein Versteifungsmodul an der Schaftwand 2 mit einer motorisch angetriebenen Verstelleinrichtung als Aktuator 15 gezeigt, an der ein Verstellelement 120 über einen Kurbeltrieb gelagert ist. Je nach Drehrichtung ist es möglich, das Verstellelement 120 in Richtung auf den proximalen Schaftrand oder entgegengesetzt in distaler Richtung zu verlagern. An dem Verstellelement 120 ist ein Versteifungselement 40 angeordnet, das beispielsweise in einer Führung verschieblich an der Schaftwand 2 gelagert ist. Der proximale Randbereich 9 des Schaftes ist grundsätzlich nachgiebig ausgebildet und kann durch Verschieben des Versteifungselementes 20 nach oben versteift werden, sodass eine erhöhte Biegesteifigkeit bereitgestellt wird. Das Versteifungsmodul kann auswechselbar an der Schaftwand 2 befestigt sein. Alternativ kann das Versteifungsmodul auch ohne Verstelleinrichtung 15 mit dem verschieblichen Verstellelement 120 und dem verschieblichen Versteifungselement 40 fest an der Außenseite oder innerhalb der Schaftwand 2 angeordnet sein. Durch Aktivieren des Aktuators und Drehen der Verstelleinrichtung 15 wird über den Kurbeltrieb das Verstellelement 120 und das Versteifungselement 40 verlagert und die Steifigkeit des fest angeordneten Versteifungsmodules in diesem Bereich verändert. Zusätzlich zu der Verstelleinrichtung 15 mit dem Versteifungselement 40, das in dem Ausführungsbeispiel verschiebbar entlang der Außenwand des Prothesenschaftes gelagert ist, ist in der Figur 7 eine Steuerungseinheit 16 dargestellt, die mit zumindest einem Sensor 14 gekoppelt ist. Die Kopplung kann drahtlos erfolgen. Alternativ oder ergänzend ist eine Kopplung des Sensors 14 oder der Sensoren 14 über ein Kabel oder über eine andere leitende Verbindung möglich und vorgesehen. Innerhalb der Steuerungseinrichtung 16 sind die notwendigen Komponenten zur Verarbeitung der Sensorsignale untergebracht, insbesondere eine Speicherkomponente 161, eine Datenverarbeitungseinrichtung 162 sowie eine Energieübertragungs- und Kommunikationsschnittstelle 17. Die Schnittstelle 17 kann Daten und/oder Energie an externe Einrichtungen senden oder von dieser empfangen und zur Verarbeitung der Datenverarbeitungseinrichtung 162, beispielsweise einem Prozessor bzw. einem Energiespeicher zuleiten. Darüber hinaus sind auch die anderen, notwendigen Komponenten in der Steuerungseinrichtung 16 integriert oder damit gekoppelt, beispielsweise ein Energiespeicher, gegebenenfalls Verstärker oder andere elektrische, elektronische oder elektromechanische Komponenten.

Von der Steuerungseinrichtung 16 werden die notwendigen Aktivierungssignale und/oder Deaktivierungssignale der Verstelleinrichtung 15 übermittelt, die in dem dargestellten Ausführungsbeispiel als ein Elektromotor ausgebildet ist. Die Übermittlung kann auch über die Kommunikationsschnittstelle 17 erfolgen. Der Aktuator 15 verlagert beispielsweise eine Drehscheibe der Verstelleinrichtung in die eine oder andere Richtung, sodass das Verstellelement 120 in Gestalt einer Schubstange das Versteifungselement 40 in die eine oder andere Richtung verlagert, um eine Veränderung der Steifigkeit eines Bereiches 9 der Schaftwand 2 zu bewirken. Das Versteifungsmodul weist ein Gehäuse auf, das über Schrauben oder Nieten oder andere Befestigungselemente an der Außenseite der Schaftwand 2 reversibel festlegbar ist. Das Versteifungsmodul kann auch in der Schaftwand 2 eingebettet werden oder in der Schaftwand 2 integriert sein. Das Gehäuse des Versteifungsmoduls dient gleichzeitig als Führung für das Versteifungselement 40, das in dem dargestellten Ausführungsbeispiel als eine längsverschiebliche Versteifungskomponente, beispielsweise als ein Schieber, eine verformbare Leiste oder eine Feder, ausgebildet ist.

In der Figur 8 ist eine weitere Ausführungsform der mechatronischen Komponente 10 in Gestalt eines Prothesenschaftes 1 dargestellt, allerdings ohne die zur Einstellung der Steifigkeit in dem proximalem Randbereich 9 notwendigen und oben beispielhaft beschriebenen Komponenten wie Sensor 14, Aktuator 15, Steuerungseinheit 16 und dergleichen. Der Prothesenschaft 10 weist einen geschlossenen distalen Endbereich 4 mit einer umlaufenden Schaftwand 2 auf. Der distale Endbereich 4 und die durchgängige Schaftwand 2 bilden einen wesentlichen starren, hülsenförmigen Prothesenschaft 10 aus, in dessen proximalem Randbereich 9 von dem proximalen Schaftrand in distale Richtung verlaufende Ausnehmungen 240 bereichsweise eingebracht sind. Der Prothesenschaft 1 ist in insgesamt fünf Sektoren 250 unterteilt, wobei ein erster, lateraler Sektor 250 L keine Ausnehmungen 240 aufweist. Die anderen vier Sektoren sind ein anteriorer Sektor 250 A, ein medialer Sektor 250 M, ein posteriorer Sektor 250 P sowie ein Sektor 250 R für die Ramusanlage. In dem dargestellten Ausführungsbeispiel weisen bis auf den lateralen Sektor 250 L alle anderen Sektoren 250 A, 250 M, 250 R und 250 P Ausnehmungen 240 auf. Die Ausnehmungen 240 können sektorweise unterschiedlich breit, in einer unterschiedlichen Dichte und/oder in unterschiedlichen Längen ausgebildet sein. Je länger die Ausnehmungen 240 sich in distale Richtung erstrecken, desto nachgiebiger ist der proximale Randbereich 9 in diesem Sektor. Gleiches gilt für eine größere Breite der Ausnehmungen 240 sowie eine erhöhte oder hohe Anzahl an Ausnehmungen 240 pro Umfangslänge. Da der laterale Sektor 250 L hauptsächlich für die seitliche Führung und Stabilität zuständig ist und eine einstellbare Nachgiebigkeit oder Elastizität aufgrund der üblichen Bewegungen mit einem Prothesenschaft nicht notwendig ist, sind in den meisten Fällen keine Ausnehmungen in dem lateralen Sektor 250 L notwendig. Alternativ oder ergänzend zu den Ausnehmungen 240 können unterschiedliche Materialstärken in den einzelnen Sektoren vorhanden sein, um die Nachgiebigkeit oder Elastizität der Schafteintrittsebene oder in dem proximalen Randbereich 9 einstellen zu können. In der rechten unteren Darstellung der Figur 8 ist eine Draufsicht auf den Prothesenschaft mit den einzelnen Sektoren 250 dargestellt. Die Ausrichtung und Benennung ergibt sich aus der Gehrichtung. In dem dargestellten Ausführungsbeispiel ist ein Prothesenschaft 10 für einen linken Stumpf dargestellt.

Die einzelnen Sektoren 250 A, 250 M, 250 R, 250 P und gegebenenfalls 250 L sind individuell in ihrer Steifigkeit veränderbar ausgebildet. Dabei ist es besonders vorteilhaft, nur den proximalen Schaftwandbereich 9 nachgiebig und schaltbar hinsichtlich der Steifigkeit auszugestalten, um in dem übrigen Bereich eine ausreichende Rigidität und Stabilität aufzuweisen. Die Einteilung und Dimensionierung der jeweiligen Sektoren kann individuell erfolgen. Jedem Sektor 250 kann ein Versteifungselement 40 mit einem Aktuator 15 zugeordnet sein, der über eine oder mehrere Steuerungseinheiten 16 unabhängig von einem anderen Sektor hinsichtlich der Steifigkeit gegen eine radiale Belastung automatisch auf der Grundlage von Sensordaten und auch manuell eingestellt werden kann.

Eine vollständige Versteifung oder eine maximale Versteifung der einzelnen Sektoren ergibt sich, wenn alle Versteifungselemente maximal aktiviert sind, sodass sich ein vollständig oder maximal versteifter Prothesenschaft in dem proximalen Randbereich ergibt. Bei einer Verringerung der Steifigkeit in dem anterioren Sektor 250 A ist die Rigidität in diesem Bereich verringert. Um den Stumpf weiterhin auch in diesem Bereich zu führen und bei einer Umschaltung von einer erhöhten Nachgiebigkeit auf eine erhöhte Steifigkeit zu verhindern, dass ein Teil oder ein Bereich des Stumpfes eingeklemmt wird, sind die zu versteifenden Sektoren oder der zu versteifende proximale Randbereich mit einem Innenschaft 260 versehen, der nachgiebig ausgebildet und in der Figur 7 eingezeichnet ist. Der Innenschaft 260 kann becherförmig mit einem distalen geschlossenen Bereich ausgebildet sein oder nur in dem Bereich des proximalen Randbereiches 9 an der Schaftwand 2 angeordnet sein. Eine erhöhte Nachgiebigkeit in dem hinteren Bereich wird durch eine Verringerung der Steifigkeit in dem posteriorer Sektor 250 P erzeugt, bei der Veränderung der Nachgiebigkeit in dem medialen Sektor 250 M werden die entsprechenden Versteifungselemente durch die Verstelleinrichtung in diesem Sektor entsprechend aktiviert oder deaktiviert, korrespondierend dazu erfolgt eine vollständige Freigabe oder eine maximale Verringerung der Steifigkeit durch Veränderung oder selektives Schalten der Steifigkeit in der Ramusanlage in dem Sektor 250 R.

Sind mehrere Sektoren nachgiebig geschaltet, beispielsweise der anteriore Sektor 250 A, der mediale Sektor 250 M, der Sektor 250 R für die Ramusanlage und der posteriorer Sektor 250 P, ist eine maximale Nachgiebigkeit des Prothesenschaftes 1 vorhanden, zumindest in dem proximalen Randbereich 9, da nur noch der laterale Sektor 250 L in dem proximalen Randbereich die ursprüngliche, maximale Steifigkeit aufweist. Ebenso ist es möglich, eine Verringerung der Steifigkeit in drei Sektoren, beispielsweise dem anterioren Sektor 250 A, dem medialen Sektor 250 M und dem Sektor 250 R für die Ramusanlage durchzuführen. Alternativ können nur zwei Sektoren hinsichtlich der Steifigkeit selektiv verändert oder verringert werden, beispielsweise der mediale Sektor M 250 in Verbindung mit dem posterioren Sektor 250 P, der mediale Sektor 250 M mit dem Sektor 250 R für die Ramusanlage oder für eine seitliche Einfassung der anteriore Sektor 250 A und der posteriore Sektor 250 P.

Die Einstellung der Steifigkeit in dem jeweiligen Bereich kann für jede Person unterschiedlich sein, neben einer rein binären Umschaltung zwischen "steif" und "nachgiebig" können unterschiedliche Steifigkeiten innerhalb der Sektoren für unterschiedliche Gangsituationen oder Nutzungssituationen vorgesehen werden. Beim Sitzen und beim Stehen ist beispielsweise vorgesehen, dass bis auf den lateralen Sektor 250 L, der in dem Ausführungsbeispiel nicht einstellbar ist, alle anderen Sektoren mit einer verringerten Steifigkeit eingestellt sind, beim Gehen oder schnellen Gehen in der Ebene ist in den vier anderen Sektoren eine erhöhte oder maximale Steifigkeit vorhanden, ebenso beim Gehen auf unebenem Gelände. Für das Gehen von Kurven ist der mediale Sektor 250 M nachgiebig geschaltet, beim Bergaufgehen sind wieder alle Sektoren versteift, beim Bergabgehen ebenso wie beim Treppe aufwärts Steigen ist der posteriore Sektor 250 P weich geschaltet, beim Treppe abwärts Gehen sind wiederum alle schaltbaren oder einstellbaren Sektoren mit einer verringerten Nachgiebigkeit oder einer erhöhten Steifigkeit versehen.

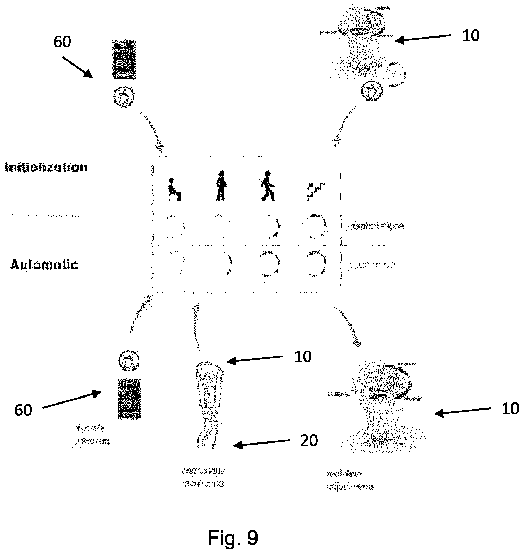

Die Einstellung der unterschiedlichen Modi oder Steifigkeiten in dem Bereich der Schafteintrittsebene in dem proximalen Randbereich 9 ist in der Figur 9 beispielhaft gezeigt. Für vier Situationen, beispielsweise das Sitzen, das Stehen, das Gehen sowie das Treppe aufwärts Gehen sind zwei unterschiedliche Modi vorgesehen, einmal den Komfortmodus, der in der oberen Zeile dargestellt ist, und der darunter dargestellte Sportmodus. Beide Modi können manuell über eine Eingabevorrichtung 60 verändert werden, sodass für jeden Patienten in jeder Situation die als angenehm empfundene Einstellung vorhanden ist. Eine Umschaltung zwischen einem Komfortmodus und dem Sportmodus kann automatisch oder durch manuelle Umschaltung mit der Eingabevorrichtung 60 erfolgen. Die Bewegungen des Nutzers werden kontinuierlich über die Sensoren überwacht, um automatisch Änderungen in dem Nutzungsverhalten oder die unterschiedlichen Bewegungssituationen zu erkennen. Die Überwachung erfolgt über Sensoren, die sowohl in dem Prothesenschaft 10 als auch in der anderen prothetischen mechatronischen Komponente 20 oder auch nur in den anderen prothetischen Komponenten vorhanden sein können. Die Einstellung der Steifigkeit der Sektoren in dem proximalen Randbereich oder auch der Steifigkeit des gesamten proximalen Randbereiches erfolgt vorteilhafterweise in Echtzeit, um stets eine angepasste Steifigkeit des Prothesenschaftes bereitstellen zu können.

## Patentansprüche

1. Verfahren zur Steuerung eines prothetischen und/oder orthetischen Systems mit einer ersten mechatronischen Komponente (10) und zumindest einer zweiten mechatronischen Komponente (20, 30), die miteinander verbunden sind, jede mechatronische Komponente weist zumindest einen Aktuator (15, 25, 35); und eine Steuerungseinheit (16, 26, 36) auf, die Steuerungseinheit (16, 26, 36) weist eine Steuerungssoftware auf, um den Aktuator (15, 25, 35) in Abhängigkeit von Sensordaten zu aktivieren, modulieren oder zu deaktivieren, wobei Sensordaten von zumindest einem Sensor (14, 24, 34) an zumindest einer der mechatronischen Komponenten (10, 20, 30) zumindest einer der Steuerungseinheiten (16, 26, 36) übermittelt und zur Steuerung des jeweiligen Aktuators (15, 25, 35) durch die Steuerungssoftware verwendet werden und die Steuerungssoftware der mechatronischen Komponenten (10, 20, 30) miteinander kommuniziert, **dadurch gekennzeichnet, dass** anhand der Sensordaten ein Zustand oder Bewegungsmuster des prothetischen und/oder orthetischen Systems erkannt und an zumindest eine weitere Steuerungseinheit (16, 26, 36) übermittelt wird und die zumindest eine weitere Steuerungseinheit (16, 26, 36) und die dazugehörige mechatronische Komponente (10, 20, 30) in eine Grundkonfiguration gebracht werden.

2. Verfahren nach Anspruch 1, **dadurch gekennzeichnet, dass** der Zustand oder das Bewegungsmuster des prothetischen und/oder orthetischen Systems von einer zentralen Steuerungseinheit (16, 26, 36) erkannt wird, die mit den anderen Steuerungseinheiten (16, 26, 36) verbunden ist.

3. Verfahren nach Anspruch 1 oder 2, **dadurch gekennzeichnet, dass** ausgehend von der Grundkonfiguration für den jeweiligen Zustand oder das Bewegungsmuster die weitere Steuerung dezentral über die den mechatronischen Komponenten (10, 20, 30) zugeordneten Steuerungseinheiten (16, 26, 36) erfolgt.

4. Verfahren nach Anspruch 2, **dadurch gekennzeichnet, dass** nach Erkennung eines vorbestimmten Zustandes oder Bewegungsmusters die weitere Steuerung der Aktuatoren der mechatronischen Komponenten (10, 20, 30) über die zentrale Steuerungseinheit (16, 26, 36) erfolgt, bis ein anderer Zustand oder ein anderes Bewegungsmuster erkannt wird.

5. Verfahren nach Anspruch 2, **dadurch gekennzeichnet, dass** die zentrale Steuerungseinheit (16, 26, 36) mit mehreren, insbesondere allen Sensoren (14, 24, 34) verbunden ist.

6. Verfahren nach einem der voranstehenden Ansprüche, **dadurch gekennzeichnet, dass** die Sensordaten drahtlos oder über Kabel zwischen den mechatronischen Komponenten (10, 20, 30) ausgetauscht werden.

7. Verfahren nach einem der voranstehenden Ansprüche, **dadurch gekennzeichnet, dass** auf der Grundlage der Sensordaten eine Steuerungseinheit (16, 26, 36) als zentrale Steuerungseinheit (16, 26, 36) zur Erkennung des Zustandes oder Bewegungsmusters bestimmt wird.

8. Verfahren nach einem der voranstehenden Ansprüche, **dadurch gekennzeichnet, dass** alle mechatronischen Komponenten (10, 20, 30) als autonom funktionierenden Komponenten (10, 20, 30) ausgebildet sind und zumindest einen Sensor (14, 24, 34) , einen Aktuator (15, 25, 35), einen Energiespeicher, eine Schnittstelle und eine Steuerungseinheit (16, 26, 36) aufweisen und die Steuerungseinheit (16, 26, 36) eine Steuerungssoftware aufweist, um den Aktuator (15, 25, 35) in Abhängigkeit von Sensordaten zu aktivieren, zu modulieren oder zu deaktivieren.

9. Verfahren nach einem der voranstehenden Ansprüche, **dadurch gekennzeichnet, dass** eine mechatronische Komponente (10) ein orthopädietechnischer Schaft ist, der eine Schaftwand aufweist, deren Steifigkeit zumindest bereichsweise reversibel über zumindest einen Aktuator (15) in Abhängigkeit von den Sensordaten verändert wird.

10. Verfahren nach Anspruch 9, **dadurch gekennzeichnet, dass** die Steifigkeit während der Benutzung des Schaftes in Echtzeit verändert wird.

11. Prothetisches und/oder orthetisches System zur Durchführung des Verfahrens nach einem der voranstehenden Ansprüche mit einer ersten mechatronischen Komponente (10) und zumindest einer zweiten mechatronischen Komponente (20, 30), die miteinander verbunden sind, wobei jede mechatronische Komponente (10, 20, 30) zumindest einen Aktuator (15, 25, 35) und eine Steuerungseinheit (16, 26, 36) aufweist, die Steuerungseinheit (16, 26, 36) weist eine Steuerungssoftware auf, um den Aktuator (15, 25, 35) in Abhängigkeit von Sensorwerten zu aktivieren, modulieren oder zu deaktivieren, wobei Sensordaten von zumindest einem Sensor (14, 24, 34) an zumindest einer der Komponenten (10, 20, 30) einer der Steuerungseinheiten (16, 26, 36) übermittelt und zur Steuerung des jeweiligen Aktuators (15, 25, 35) durch die Steuerungssoftware verwendet werden und die Steuerungssoftware der Komponenten (10, 20, 30) miteinander kommuniziert, **dadurch gekennzeichnet, dass** die Steuerungssoftware eingerichtet ist, anhand der Sensordaten einen Zustand oder ein Bewegungsmuster des prothetischen und/oder orthetischen Systems zu erkennen und an alle Steuerungseinheiten (16, 26, 36) zu übermitteln und zumindest einer weitere Steuerungseinheit (16, 26, 36) oder alle Steuerungseinheiten (16, 26, 36) und die dazugehörigen Komponenten (10, 20, 30) in eine Grundkonfiguration zu bringen.

12. Prothetisches und/oder orthetisches System nach Anspruch 11, **dadurch gekennzeichnet, dass** die mechatronischen Komponenten (10, 20, 30) Befestigungseinrichtungen zum lösbaren Festlegen aneinander aufweisen.

13. Prothetisches und/oder orthetisches System nach Anspruch 11 oder 12, **dadurch gekennzeichnet, dass** jede mechatronische Komponente (10, 20, 30) eine eigene Schnittstelle (17, 27, 37) zum Transport von Energie und/oder Daten aufweist.

14. Prothetisches und/oder orthetisches System nach einem der Ansprüche 11 bis 13, **dadurch gekennzeichnet, dass** eine mechatronische Komponente (10) als orthopädietechnischer Schaft ausgebildet ist und zumindest eine Schaftwand (2) aufweist, an der zumindest ein Versteifungselement (40) angeordnet ist, über das die Steifigkeit der Schaftwand (2) zumindest bereichsweise reversibel veränderbar ist.

15. Prothetisches und/oder orthetisches System nach Anspruch 14, **dadurch gekennzeichnet, dass** die Schaftwand (2) Sektoren aufweist, die über den Schaftumfang verteilt sind und deren Steifigkeit unabhängig voneinander reversibel veränderbar ist.

16. Prothetisches und/oder orthetisches System nach einem der Ansprüche 11 bis 14, **dadurch gekennzeichnet, dass** eine erste mechatronische Komponente (10) ein künstliches Kniegelenk (12) mit einem Oberteil (28) und einem Unterteil (29) ist, die schwenkbar aneinander gelagert sind und an denen der Aktuator (25) angeordnet ist und dass die zweite mechatronische Komponente (20, 30) ein künstliches Hüftgelenk, ein Oberschenkelschaft, eine Befestigungseinrichtung und/oder ein künstliches Knöchelgelenk (23) ist.

17. Prothetisches und/oder orthetisches System nach Anspruch 16, **dadurch gekennzeichnet, dass** die erste mechatronische Komponente (10) als Master-Komponente und die zumindest eine zweite mechatronische Komponente (20, 30) als Slave-Komponente ausgebildet ist.

## Claims

1. A method for controlling a prosthetic and/or orthotic system having a first mechatronic component (10) and at least one second mechatronic component (20, 30), which are connected to one another, each mechatronic component having at least one actuator (15, 25, 35); and a control unit (16, 26, 36), the control unit (16, 26, 36) having control software to activate, modulate or deactivate the actuator (15, 25, 35) as a function of sensor data, wherein sensor data are transmitted from at least one sensor (14, 24, 34) on at least one of the mechatronic components (10, 20, 30) to at least one of the control units (16, 26, 36) and are used for the control of the respective actuator (15, 25, 35) by the control software and the control software of the mechatronic components (10, 20, 30) communicates with one another,
**characterized in that** a status or movement pattern of the prosthetic and/or orthotic system is recognized on the basis of the sensor data and transmitted to at least one further control unit (16, 26, 36) and the at least one further control unit (16, 26, 36) and the associated mechatronic component (10, 20, 30) are brought into a base configuration.

2. The method as claimed in claim 1, **characterized in that** the status or the movement pattern of the prosthetic and/or orthotic system is recognized by a central control unit (16, 26, 36) which is connected to the other control units (16, 26, 36).

3. The method as claimed in claim 1 or 2, **characterized in that**, starting from the base configuration for the respective status or the movement pattern, the further control is carried out in a decentralized manner via the control units (16, 26, 36) assigned to the mechatronic components (10, 20, 30).

4. The method as claimed in claim 2, **characterized in that.** after recognition of a predetermined status or movement pattern, the further control of the actuators of the mechatronic components (10, 20, 30) is carried out via the central control unit (16, 26, 36) until another status or another movement pattern is recognized.

5. The method as claimed in claim 2, **characterized in that** the central control unit (16, 26, 36) is connected to multiple, in particular all sensors (14, 24, 34).

6. The method as claimed in any one of the preceding claims, **characterized in that** the sensor data are exchanged wirelessly or via wire between the mechatronic components (10, 20, 30).

7. The method as claimed in any one of the preceding claims, **characterized in that**, on the basis of the sensor data, one control unit (16, 26, 36) is determined as the central control unit (16, 26, 36) for recognizing the status or movement pattern.

8. The method as claimed in any one of the preceding claims, **characterized in that** all mechatronic components (10, 20, 30) are designed as autonomously functioning components (10, 20, 30) and have at least one sensor (14, 24, 34), an actuator (15, 25, 35), an energy storage device, an interface, and a control unit (16, 26, 36), and the control unit (16, 26, 36) has control software to activate, modulate or deactivate the actuator (15, 25, 35) as a function of sensor data.

9. The method as claimed in any one of the preceding claims, **characterized in that** a mechatronic component (10) is an orthopedic socket, which has a socket wall, the stiffness of which is changed at least in some areas reversibly via at least one actuator (15) as a function of the sensor data.

10. The method as claimed in claim 9, **characterized in that** the stiffness is changed during the use of the socket in real time.

11. A prosthetic and/or orthotic system for carrying out the method as claimed in any one of the preceding claims, having a first mechatronic component (10) and at least one second mechatronic component (20, 30), which are connected to one another, wherein each mechatronic component (10, 20, 30) has at least one actuator (15, 25, 35) and a control unit (16, 26, 36), the control unit (16, 26, 36) has control software to activate, modulate or deactivate the actuator (15, 25, 35) as a function of sensor data, wherein sensor data are transmitted from at least one sensor (14, 24, 34) on at least one of the components (10, 20, 30) to one of the control units (16, 26, 36) and are used for the control of the respective actuator (15, 25, 35) by the control software and the control software of the components (10, 20, 30) communicates with one another, **characterized in that** the control software is configured to recognize a status or movement pattern of the prosthetic and/or orthotic system on the basis of the sensor data and transmit it to all the control unit (16, 26, 36) and to bring at least one further control unit (16, 26, 36) or all the control units (16, 26, 36) and the associated components (10, 20, 30) into a base configuration.

12. The prosthetic and/or orthotic system as claimed in claim 11, **characterized in that** the mechatronic components (10, 20, 30) have fastening units for detachable securing on one another.

13. The prosthetic and/or orthotic system as claimed in claim 11 or 12, **characterized in that** each mechatronic component (10, 20, 30) has a separate interface (17, 27, 37) for the transport of energy and/or data.

14. The prosthetic and/or orthotic system as claimed in any one of claims 11 to 13, **characterized in that** a mechatronic component (10) is designed as an orthopedic socket and has at least one socket wall (2), on which at least one stiffening element (40) is arranged, via which the stiffness of the socket wall (2) is reversibly changeable at least in some areas.

15. The prosthetic and/orthotic system as claimed in claim 14, **characterized in that** the socket wall (2) has sectors, which are distributed over the socket circumference and the stiffness of which is reversibly changeable independently of one another.

16. The prosthetic and/or orthotic system as claimed in any one of claims 11 to 14, **characterized in that** a first mechatronic component (10) is an artificial knee joint (12) having an upper part (28) and a lower part (29), which are pivotably mounted on one another and on which the actuator (25) is arranged, and **in that** the second mechatronic component (20, 30) is an artificial hip joint, a thigh socket, a fastening unit, and/or an artificial ankle joint (23).

17. The prosthetic and/or orthotic system as claimed in claim 16, **characterized in that** the first mechatronic component (10) is designed as a master component and the at least one second mechatronic component (20, 30) is designed as a slave component.

## Revendications

1. Procédé de commande d'un système prothétique et/ou orthétique comprenant un premier composant mécatronique (10) et au moins un deuxième composant mécatronique (20, 30) qui sont reliés entre eux, chaque composant mécatronique comportant au moins un actionneur (15, 25, 35) ; et une unité de commande (16, 26, 36), l'unité de commande (16, 26, 36) comportant un logiciel de commande pour activer, moduler ou désactiver l'actionneur (15, 25, 35) en fonction de données de capteur, les données de capteur d'au moins un capteur (14, 24, 34), situé sur l'un au moins des composants mécatroniques (10, 20, 30), étant transmises à l'une au moins des unités de commande (16, 26, 36) et étant utilisées pour commander l'actionneur respectif (15, 25, 35) par le logiciel de commande, et les logiciels de commande des composants mécatroniques (10, 20, 30) communiquant entre eux,
**caractérisé en ce qu'**un état ou un modèle de mouvement du système prothétique et/ou orthétique est détecté à l'aide des données de capteur et est transmis à au moins une autre unité de commande (16, 26, 36), et ladite au moins une autre unité de commande (16, 26, 36) et le composant mécatronique associé (10, 20, 30) sont mis dans une configuration de base.

2. Procédé selon la revendication 1,
**caractérisé en ce que** l'état ou le modèle de mouvement du système prothétique et/ou orthétique est détecté par une unité de commande centrale (16, 26, 36) qui est reliée aux autres unités de commande (16, 26, 36).

3. Procédé selon la revendication 1 ou 2,
**caractérisé en ce que**, à partir de la configuration de base pour l'état ou le modèle de mouvement respectif, la commande ultérieure s'effectue de manière décentralisée via les unités de commande (16, 26, 36) associées aux composants mécatroniques (10, 20, 30).

4. Procédé selon la revendication 2,
**caractérisé en ce qu'**après la détection d'un état ou d'un modèle de mouvement prédéfini, la commande ultérieure des actionneurs des composants mécatroniques (10, 20, 30) s'effectue via l'unité de commande centrale (16, 26, 36) jusqu'à ce qu'un état différent ou un modèle de mouvement différent soit détecté.

5. Procédé selon la revendication 2,
**caractérisé en ce que** l'unité de commande centrale (16, 26, 36) est reliée à plusieurs capteurs, en particulier à tous les capteurs (14, 24, 34).

6. Procédé selon l'une des revendications précédentes,
**caractérisé en ce que** les données de capteur sont échangées sans fil ou par câble entre les composants mécatroniques (10, 20, 30).

7. Procédé selon l'une des revendications précédentes,
**caractérisé en ce que**, sur la base des données de capteur, une unité de commande (16, 26, 36) est désignée comme unité de commande centrale (16, 26, 36) pour la détection de l'état ou du modèle de mouvement.

8. Procédé selon l'une des revendications précédentes,
**caractérisé en ce que** tous les composants mécatroniques (10, 20, 30) sont conçus comme des composants fonctionnant de manière autonome (10, 20, 30) et comportent au moins un capteur (14, 24, 34), un actionneur (15, 25, 35), un accumulateur d'énergie, une interface et une unité de commande (16, 26, 36), et l'unité de commande (16, 26, 36) comporte un logiciel de commande pour activer, moduler ou désactiver l'actionneur (15, 25, 35) en fonction des données de capteur.

9. Procédé selon l'une des revendications précédentes,
**caractérisé en ce qu'**un composant mécatronique (10) est une emboîture orthopédique qui comprend une paroi d'emboîture dont la rigidité est modifiée de manière réversible au moins localement au moyen d'au moins un actionneur (15) en fonction des données de capteur.

10. Procédé selon la revendication 9,
**caractérisé en ce que** la rigidité est modifiée en temps réel pendant l'utilisation de l'emboîture.

11. Système prothétique et/ou orthétique pour la mise en œuvre du procédé selon l'une des revendications précédentes, comprenant un premier composant mécatronique (10) et au moins un deuxième composant mécatronique (20, 30) qui sont reliés entre eux, chaque composant mécatronique (10, 20, 30) comportant au moins un actionneur (15, 25, 35) et une unité de commande (16, 26, 36), l'unité de commande (16, 26, 36) comportant un logiciel de commande pour activer, moduler ou désactiver l'actionneur (15, 25, 35) en fonction de valeurs de capteur, les données de capteur d'au moins un capteur (14, 24, 34), situé sur l'un au moins des composants (10, 20, 30), étant transmises à l'une des unités de commande (16, 26, 36) et étant utilisées pour commander l'actionneur respectif (15, 25, 35) par le logiciel de commande, et les logiciels de commande des composants (10, 20, 30) communiquant entre eux,
**caractérisé en ce que** le logiciel de commande est conçu pour détecter, à l'aide des données de capteur, un état ou un modèle de mouvement du système prothétique et/ou orthétique et pour le transmettre à toutes les unités de commande (16, 26, 36) et pour mettre dans une configuration de base au moins une autre unité de commande (16, 26, 36) ou toutes les unités de commande (16, 26, 36) et les composants associés (10, 20, 30).

12. Système prothétique et/ou orthétique selon la revendication 11,
**caractérisé en ce que** les composants mécatroniques (10, 20, 30) comportent des dispositifs de fixation destinés à les fixer les uns aux autres de manière amovible.

13. Système prothétique et/ou orthétique selon la revendication 11 ou 12,
**caractérisé en ce que** chaque composant mécatronique (10, 20, 30) comporte une interface propre (17, 27, 37) pour le transport d'énergie et/ou de données.

14. Système prothétique et/ou orthétique selon l'une des revendications 11 à 13,
**caractérisé en ce qu'**un composant mécatronique (10) est conçu comme une emboîture orthopédique et comporte au moins une paroi d'emboîture (2) sur laquelle est disposé au moins un élément de renfort (40) permettant de modifier de manière réversible au moins localement la rigidité de la paroi d'emboîture (2).

15. Système prothétique et/ou orthétique selon la revendication 14,
**caractérisé en ce que** la paroi d'emboîture (2) comporte des secteurs qui sont répartis sur la circonférence de l'emboîture et dont la rigidité peut être modifiée de manière réversible indépendamment les uns des autres.

16. Système prothétique et/ou orthétique selon l'une des revendications 11 à 14,
**caractérisé en ce qu'**un premier composant mécatronique (10) est une articulation de genou artificielle (12) munie d'une partie supérieure (28) et d'une partie inférieure (29) qui sont montées l'une sur l'autre de manière à pouvoir pivoter et sur lesquelles est disposé l'actionneur (25), et **en ce que** le deuxième composant mécatronique (20, 30) est une articulation de hanche artificielle, une tige fémorale, un dispositif de fixation et/ou une articulation de cheville artificielle (23).

17. Système prothétique et/ou orthétique selon la revendication 16,
**caractérisé en ce que** le premier composant mécatronique (10) est conçu comme composant maître et ledit au moins un deuxième composant mécatronique (20, 30) est conçu comme composant esclave.
